# EUROPEAN PATENT SPECIFICATION

(11) **EP 1 318 801 B1**
(45) Date of publication and mention of the grant of the patent: **11.03.2009**
(21) Application number: 01973897.0
(22) Date of filing: 19.09.2001
(51) Int. Cl.: A61K 31/13, A61K 31/131, A61P 29/00

(54) **TOPICAL ANALGESIC COMPOSITIONS CONTAINING ALIPHATIC POLYAMINES AND METHODS OF USING SAME**
TOPISCHE ANALGETISCHE ZUSAMMENSETZUNGEN ENTHALTEND ALIPHATISCHE POLYAMINE UND METHODEN FÜR IHRE VERWENDUNG
COMPOSITIONS ANALGESIQUES TOPIQUES CONTENANT DES POLYAMINES ALIPHATIQUES ET MODALITES D'UTILISATION

(30) Priority: 19.09.2000 US 233488 P
(43) Date of publication of application: 18.06.2003
(73) Proprietor: Dermal Therapy (Barbados) Inc., Christ Church (BB)
(72) Inventor: PERLMUTTER, Alan, L., London, Ontario N5X 2N9 (CA); WICKETT, R., Randall, Cincinnati, OH 45255 (US)
(74) Representative: Vossius & Partner
(86) International application number: PCT/CA2001/001337
(87) International publication number: WO 2002/024185

(56) References cited:
- FR-A- 2 775 901
- GENEDANI S ET AL: "PUTRESCINE HAS ANALGESIC ACTIVITY IN RATS" LIFE SCIENCES, vol. 34, no. 24, 1984, pages 2407-2412, XP001080369 ISSN: 0024-3205
- TSAI Y-H ET AL: "PERCUTANEOUS ABSORPTION OF PIROXICAM FROM OINTMENT BASES IN RABBITS" INTERNATIONAL JOURNAL OF PHARMACEUTICS (AMSTERDAM), vol. 24, no. 1, 1985, pages 61-78, XP001080377 ISSN: 0378-5173
- ONAL A ET AL: "Agmatine produces antinociception in tonic pain in mice." PHARMACOLOGY, BIOCHEMISTRY, AND BEHAVIOR. UNITED STATES 2001 MAY-JUN, vol. 69, no. 1-2, May 2001 (2001-05), pages 93-97, XP001079156 ISSN: 0091-3057

## Description

This invention relates to topical analgesia and to uses and compositions, etc. for treating pain or discomfort in a mammal.

Pain occurs frequently and is a common symptom for which patients seek medical assistance. Defined as an unpleasant subjective sensation which results from a noxious stimulus, pain alerts the body to possible or actual danger, such as during disease or physical trauma. The needs of a patient in temporary or chronic pain include comfort, freedom from adverse reactions, the ability to perform the functional activities of daily living, psychological reassurance, and a satisfying quality of life.

The pathological mechanic of pain and its perception by the subject remains an area of considerable research. Despite a lack of comprehensive understanding of the many dimensions of pain, many agents have been developed to be effective in its treatment.

Analgesics are a broad class of agents developed for use in the treatment and management of pain. Major classes of analgesic compounds include analgesic-antipyretic compounds which are compounds that alleviate pain and/or reduce fever, such as salicylates and related compounds, and narcotic analgesics or opiates, which alleviate pain and/or induce sleep. The analgesic potency of a compound generally correlates with its solubility in lipids. It is believed that analgesia occurs when lipid structures in neurosensory cell membranes are disrupted by a dissolved analgesic agent.

Analgesics can be broadly divided into two classes of agents, i.e: systemic analgesics and topical analgesics. Compounds displaying analgesic properties are not necessarily effective as both systemic and topical analgesics. Systemic analgesic, which are typically swallowed or injected, are frequently prescribed for the treatment of pain. The most common treatment for chronic pain is with the use of the salicylate-like agents known as nonsteroidal anti-inflammatory drugs (NSAIDs). Unfortunately, side effects can be associated with the use of these drugs, including gastrointestinal and renal abnormalities.

Unlike systemic agents that are swallowed or injected, topical analgesics, typically available in the form of salves, creams, ointments and rubs, work only on the area they are rubbed into, reducing or eliminating the risk of systemic side effects. Topical analgesics may be appropriate for subjects with muscle ache or mild pain that affects only a few joints. They may also provide relief for subjects whose oral medications alone fail to reduce their pain to manageable levels, such as, for example, from arthritic pain. They may also provide means of prophylaxis of pain. However, topical administration of an analgesic requires that the analgesic be able to reach the sensory receptors implicated in pain. In particular, topical analgesics for use on the skin must first be able to penetrate dense stratum corneum, keratinized corneocytes and the restrictive epidermal cell layer barrier of the skin surface.

Different types of agents have been found to be effective as topical analgesics. Most common topical analgesics include one or more of three commonly used analgesic agents which can be broadly classified as counter-irritants, salicylates, and caspaicin. Counter-irritants stimulate nerve endings distracting the brain's attention from muscoskeletal pain, and encompass such substances as menthol, camphor, etc. Salicylates inhibit prostaglandins which contribute to pain and inflammation. Such compounds are often found to act as systemic analgesics as well. Caspaicin is a naturally occurring drug which works by depleting a neurotransmitter substance that is implicated in sending pain messages to the brain. These active agents are usually applied as components in compositions to an area in need of analgesia. These compositions often include agents that aid in the transcutaneous delivery of the analgesic agent to the sensory receptors. As well, lidocaine, lignocaine, xylocaine, benzocaine, tetracine, prilocaine, bupivacaine, and the like, are used as topical analgesics. However, their toxicities are well established and great care must be taken to ensure that the dosage of these agents is not exceeded to toxic levels. Toxic effects include formation of sulfhemoglobin and methhemoglobin, as well as effects on the central nervous system.

Thus, the development of topical analgesics for the management of pain which provide fast and effective relief or reduction of pain, yet exhibit reduced side effects, is an ongoing need.

The present invention involves alleviation of pain or discomfort of a subject by aliphatic polyamines that produce an analgesic effects in their topical administration to the subject. An analgesic is an agent used in the treatment of pain.

One embodiment of the invention is a method for producing local analgesia in a subject having a site of local discomfort The method includes topically administering an effective amount of an aliphatic polyamine to the site.

In another embodiment, the invention is a method for producing relief in a subject having a site subject of nociceptive stimulation. The method includes topically administering an effective amount of an aliphatic polyamine to the site.

The polyamine can be in the form of a free base or it can be a pharmaceutically acceptable salt of a polyamine, or it can be a mixture of the two. Of course, the form selected would be compatible with the use to which the polyamine is to be put. For application to human skin, the free base or salt(s) would be compatible for use with human skin. Salts that produce deleterious effects would generally be avoided.

In particular embodiments, the polyamine has up to fifteen carbon atoms. Certain preferred polyamines are limited to four amino groups, while certain embodiments have three amino groups and some have only two amino groups. In certain embodiments, the polyamine(s) is saturated. In certain embodiments, the polyamine is non-cyclic. In certain embodiments, the polyamine has only ten carbon atoms, but it may have more, or less, particularly, nine, or eight, or seven, or six or five carbon atoms. Particular polyamines of the invention are putrescine, cadaverine, spermine and/or spermidine. Spermidine is the "doublet" of putrescine and spermine is the "triplet." Preferably, the polyamine has at least four carbon atoms.

In compositions of the invention, the polyamine can make up between 0.1 and 10 weight percent of the composition that is to be administered to the site. In other embodiments, between 0.2 and 9 percent, or between 0.3 and 8 percent, or between 0.4 and 7 percent, or between 0.5 and 6 percent, or between 0.6 and 5 percent, or between 0.7 and 5 percent, or between 0.8 and 5 percent, or between 1 and 4 percent or between 1 and 3 percent, or between 1 and 2 percent. The polyamine can thus constitute about 0.1, 0.2, 0.3, 0.4, 0.5, 0.8, 1.0, 1.3, 1.5, 1.8, 2.0, 2.3, 2.5, 2.8, 3.0, 3.5, 4.0, 4.5; 5.0, 5.5, 6, 7, 8, 9 or 10 weight percent of the composition that is to be administered to the site.

The site of topical application would usually be the epidermis, the outer skin layer of the subject. The subject is usually human, but could be another mammal, such as a horse or dog, for example.

A composition of the invention preferably includes a chaotropic agent, or penetration agent, which enhances delivery of the analgesic component into lower layers of the skin aiding its delivery to the site of action. The agent can be urea, or it can be oleic acid, for example, or it can be a combination of such agents.

Urea has been found to be particularly useful in this regard. Preferred embodiment compositions include urea in a concentration of at least 10 percent, more preferably in a concentration of at least 15 percent by weight of the composition. Usually, the concentration of urea would be no more than 40 percent The concentration of urea could be about 10, or about 15 or about 20, or about 25 or about 30 or about 35 or about 40 percent by weight of the total weight of the composition.

In certain embodiments of the invention, the composition is a cream, or a spray, or an ointment, or a gel or a lotion, for application to a subject's skin.

A preferred mode of administration is with the use of a patch. A patch can be mounted to the site to be treated. The patch has the polyamine incorporate thereinto at the site such that the polyamine is transferred from the patch to the site.

In using such a patch, release of the polyamine from the patch can be controlled to produce the analgesic effect over a period of time. The period of time can be, for example, up to about one week, or between about one hour and one week, or between one hour and six days, or between six hours and five days, or between six hours and four days, or between twenty-four hours and three days, or between one day and two days.

In certain preferred embodiments, the composition can include beta 1,3-D glucan.

In a particularly preferred embodiment, the composition includes lidocaine, a known local anaesthetic.

Topical sites treatable through the present invention include those where discomfort is the result of a sports injury, a physical assault, arthritis, rheumatism, headache, shingles, surgical pain, or a combination of any of the foregoing.

The source of discomfort can be one of non-pathological etiology.

The invention can be used in a subject suffering from fibromyalgia.

In a particular embodiment, the site of treatment is free of extracellular wound scar skin tissue and/or the site is free of a wound undergoing healing of the skin.

The invention includes manufacture of a composition of the invention, and particularly those for use according to any method of the invention, for example, in topically administering the composition to an affected site.

In preferred embodiment compositions, the polyamine of the invention is admixed with a suitable pharmaceutically-acceptable diluent or carrier.

Several properties of putrescine are known that make it suitable for use as a topical analgesic, including that it is a naturally occurring substance which is a normal metabolite in cells, it is a ubiquitous component in foods, and it has been shown to have a very low toxicity when administered via many routes including orally, intravenously, intraperitoneally, and intramuscularly.

In a preferred embodiment, the aliphatic polyamine is putrescine dihydrochloride.

In another aspect, the invention relates to a pharmaceutical composition in dosage unit form suitable for topical administration to a mammal for effecting analgesia in a mammal desiring such analgesia, which comprises an effective amount of an aliphatic polyamine or a pharmaceutically-acceptable salt thereof, in admixture with a suitable pharmaceutically acceptable diluent or carrier.

In another aspect, the invention provides a commercial package containing as an active pharmaceutical ingredient an aliphatic alkyl polyamine or a pharmaceutically-acceptable salt thereof, together with instructions for the use thereof for inducing analgesia in a mammal

In another aspect, the invention provides a process for preparing an agent for effecting topical analgesia in a mammal, in ready-to-use drug form for the treatment of pain, characterized in that an aliphatic polyamine or a pharmaceutically acceptable salt thereof is used as an active ingredient in the agent.

For purposes of clarity, the following terms and phrases used throughout this specification and the appended claims are defined in the manner set forth directly below.

The term "analgesia" as used herein means the reduction, or absence, of sensibility to pain, designating particularly the relief of pain without consciousness.

The term "malady" generally refers to an illness or disease.

The term "composition" is meant to embrace both a single substance and a mixture of substances.

The term "polyamine" as used herein means one or more than one amino group.

The term "amino" as used herein means -NH₂.

The term "aliphatic" means acyclic or cyclic, saturated or unsaturated carbon compounds, excluding aromatic compounds. Saturated carbon compounds include hydrocarbons having from one to twenty carbon atoms, within which includes from four to eleven carbon atoms, and further which includes from four to five carbons, and which can be straight or branched chain. Representatives of such groups are n-butyl, n-pentyl, n-propyl sec-butyl, isobutyl, etc.

The term "alkyl" as employed herein means a saturated hydrocarbon having from one to twenty carbon atoms, within which includes from four to eleven carbon atoms, and further which includes from four to five carbons, and which can be straight or branched chain. Representatives of such groups are n-butyl, n-pentyl, n-propyl, sec-butyl, isobutyl, etc.

The term "pharmaceutically-acceptable" as employed herein means those compounds, materials, compositions, and/or dosage forms which are, within the scope of sound medical judgement, suitable for use in contact with the tissues of human beings and animals without excessive toxicity, irritation, allergic response, or other problems or complication, commensurate with a reasonable benefit/risk ratio.

The term "pharmaceutically-acceptable salts" in this respect refers to the relatively-non-toxic, inorganic and organic addition salts of compounds of the present invention. Representative salts include the hydrochloride, hydrobromide, sulphate, phosphate, nitrate, acetate (see for example, S.M. Berge et al., "Pharmaceutical Salts", J. Pharm. Sci. 66:1-19 (1977)).

The term "chaotropic agent" as employed herein means an agent that breaks up dense macromolecular and lipid-rich domains. Some examples of materials that can serve as chaotropic agents include urea, substitutes ureas, amides and dimethyl sulphoxide.

The phrase "pharmaceutically-acceptable carrier" as employed herein means a pharmaceutically-acceptable material, composition or vehicle, as defined directly above, such as a liquid or solid filler, diluent, excipient, solvent, involved in carrying or transporting a chemical compound or pharmaceutical agent from one portion of the body to another portion of the body. Some examples of materials that can serve as carriers include: sugars, such as lactose and glucose; starches, such as corn starch; cellulose, and its derivatives, such as sodium carboxymethyl cellulose and cellulose acetate; malt; gelatin; talc; oils, such as olive oil; glycols, such as propylene glycol; polyols, such as glycerin, polyethylene glycol; ester, such as ethyl oleate; agar; buffering agents, such as magnesium hydroxide; water; ethyl alcohol; and other non-toxic compatible substances emplyed in pharmaceutical formulations.

The present invention includes a use of an aliphatic polyamine as a topical analgesic and topical analgesic compositions containing an aliphatic alkyl polyamine.

A number of the aliphatic alkyl polyamine compounds useful in the composition and methods of the present invention are known in the chemical art. The amine group contained by the aliphatic polyamines may be either primary or secondary and may be located either in a terminal position, within the alkane chain, or both. In a preferred embodiment, the preferred aliphatic polyamines useful in the compositions and methods of the present invention are spermidine (4,4'-iminobis butylamine), spermine (*N,N'*-Bis(3-aminopropyl)-1,4-butane-diamine), cadaverine (1,5-pentanediamine) and putrescine (1,4-diaminobutane). Details of the synthetic preparation of a number of the aliphatic polyamines utilizable in the compositions and methods of the present invention may be found in *Beilsteins Handbuch Der Organischen Chemie.* The *Merck Index, 11th edition,* also references many of the preferred compounds of this invention.

The free base form of the aliphatic polyamines utilized in the present invention may be conveniently converted to the corresponding acid addition salt by contacting a solution of the free base with the appropriate acid. Particularly preferred salts are the acid addition salts formed with hydrochloric and sulfuric acids, e.g., hydrochloride and sulfate.

The compositions of the present invention comprise one or more of the above-mentioned aliphatic polyamines in a sufficient quantity together with a suitable pharmaceutical carrier to induce topical analgesia. The aliphatic polyamines act as analgesic agents. A sufficient quantity is defined as the amount of compound necessary to induce analgesia. In the usual course of therapy, the aliphatic polyamine is incorporated into an acceptable vehicle to form a composition for topical administration to the area sensing pain and, thus, requiring analgesia.

The dosage levels of the aliphatic polyamine in the pharmaceutical compositions of this invention may be varied so as to obtain an amount of the active ingredient that is effective to achieve the desired therapeutic response for a particular use, or composition without being toxic to the subject. In a preferred embodiment, the amount is 1.0 weight percent. In another embodiment, the amount is 0.8 weight percent. In yet another preferred embodiment, the amount is 2.0 weight percent. In other embodiments, the percentage may be higher or lower. In a preferred embodiment, a concentration of putrescine at 0.8 percent weight per volume of the composition in a eutectic base is used for inducing analgesia to an area of the skin of a human. A topical composition containing 0.8 percent weight per volume of putrescine in a eutectic base for the treatment of scar tissue is described in United States Patent Serial No. 5,885,982. In general, such compositions are envisioned to contain the active ingredient in from about 0.005% to about 5% volume by weight of the total composition. The use of putrescine as a non-topical, systemic analgesic by intraperitoneal and intracerebroventricular injection into rats at 200 to 400 mg/kg of body weight is known. See, Genedani et al., Life Sciences, 34, 2407-2412 (1984).

While it is might be possible for an aliphatic alkyl polyamine of the present invention to be administered in pure form, it is preferable to administer the compounds as a topical pharmaceutical composition to facilitate spreading over the area in need of analgesia. Compositions for topical application may be exemplified by ointments, creams, lotions, solutions, suspensions, aerosols, gels, dusting powder, and impregnated bandages and dressings. Such compositions would normally be based upon standard carriers such as pharmaceutically acceptable vegetable oils and gelatins, gums and petrolatum. Other ingredients of the composition of the invention may be preservatives, coloring, thickening, suspending, disbursing, emulsifiing, swelling, stabilizing and buffering agents, fats, oils, waxes, paraffins, starch, polyethylene glycols, silicones, bentonites, talc, zinc oxide, etc., as required by the specific formulation. In a preferred embodiment, the pharmaceutical carrier is a eutectic base (Glaxo Canada Ltd., Toronto, Ont.).

A polyamine of the invention can also be incorporated into patches or so-called transdermal therapeutic systems (ITS). From these the active substance components act on the skin over a defined area of the body surface in occlusive manner at a controlled release rate and are appropriately brought to transdermal absorption.

It is well known that the rate of transport of some substances through the skin depends on the polar-nonpolar nature of the substance, size of the substance, hydration of the skin, blood supply, and modification of the stratum corneum by chemicals. Thus, in a preferred embodiment, a chaotropic agent is present in the composition. In a preferred embodiment, the chaotropic agent is urea. In yet another preferred embodiment, urea is present at about fifteen weight percent of the composition. In yet another preferred embodiment, urea is present from between about one and twenty weight percent of the composition. Such chaotropic agents can facilitate penetration of the analgesic agent into the skin as they break up dense macromolecular domains of fibrous and globular proteins.

In another preferred embodiment, a beta-1,3-glucan can be added to promote healing in the area of the subject requiring analgesia should the area also require healing. In another embodiment, a beta-1,3-glucan is present at about six to eight percent by weight of the total composition. Beta-1,3-glucans may be derived from, among other things, purified yeast cell walls, and are well known to stimulate the immunosystem.

Repeated use of the analgesic compositions of the present invention is envisaged, the length of time of use being dependent upon the length of time that is required until analgesia is effected. As well, the dosage size and frequency of administration can vary depending upon the nature and intensity of the pain. The exact dosage to be administered and length of time of use with a subject will, of course, be dependent upon, among other factors, the particular compositions employed, and the disease or injury being treated.

A variety of uses have been tried for the analgesic compositions of the present invention invention, for example: headache, frontal headache, arthritis, anti-nociception, rheumatism, shingles, post-herpetic neuralgia, joint pain (in the arm, leg, shoulder, toe, ankle, etc. for example), post surgical pain, tenderness in breasts, burns, tense muscles, chest pain, injuries, sports injuries (for example, shin splints, pulled muscles, sprain, etc.), repetitive stress injuries including tennis elbow, fibromyalgia, rotator cuff pain, muscoskeletal pain. In a preferred embodiment, the composition of the invention is applied to an area of a shoulder requiring analgesia due to pain originating from the rotator cuff and includes putrescine at about 2 percent by weight of the composition.

The following examples describe in detail compositions illustrative of the present invention and methods for their utilization. It will be apparent to those skilled in the art that many modifications, both of materials and methods may be practiced without departing from the purpose and intent of the disclosure. All components are commercially available.

### Example 1

Topical cream composition according to the present invention is prepared from the following components:

| Component | Percent by weight |
|---|---|
| deionized water | 55 |
| urea USP | 15 |
| glycerin | 6 |
| triethanolamine 99% | 4 |
| GMS/PEG 100 stearate | 3.5 |
| emulsifying wax NF (polawax) | 3 |
| hydrogenated polyisobutylene | 3 |
| lactic acid | 3 |
| cetyl alcohol (hexadecyl alcohol) | 2.5 |
| putrescine | 1 |
| malic acid | 3 |
| silk protein (amino acid) | 1 |
| imidazolidinyl urea | 0.4 |
| methyl paraben | 0.2 |
| carbomer 934P | 0.1 |
| propyl paraben | 0.1 |
| tetra sodium EDTA | 0.1 |

### Example 2

Topical balm composition according to the present invention is prepared from the following components:

| Component | Percent by weight |
|---|---|
| Beeswax | 5 |
| Cetyl alcohol | 3 |
| GMS/PEG 100 stearate | 6 |
| imidazolidinyl urea | 0.4 |
| Lactic acid | 3 |
| Lanolin | 4 |
| Malic acid | 3 |
| Methyl paraben | 0.2 |
| Mineral oil | 5 |
| Propyl paraben | 0.1 |
| Putrescine | 1 |
| Silk protein (amino acid) | 2 |
| Tetra sodium EDTA | 0.1 |
| Triethanolamine | 0.25 |
| Urea USP | 25 |
| Deionized water | 41.95 |

### Example 3

Topical lotion composition according to the present invention is prepared from the following components:

| Component | Percent by weight |
|---|---|
| Carbomer 941 | 0.1 |
| Cetyl alcohol | 2 |
| Emulsifying wax NF (polawax) | 1 |
| GMS/PEG 100 stearate | 2.0 |
| Hydrogenated polyisobutene | 4.5 |
| imidazolidinyl urea | 0.4 |
| Isopropyl myristate | 4 |
| Lactic acid | 3 |
| Malic acid | 2 |
| Propylene glycol | 5 |
| Putrescine | 2 |
| Quaternium 15 (Dow 200) | 0.05 |
| Silk protein | 1 |
| Tetra sodium EDTA | 0.1 |
| Triethanolamine 99% | 1.25 |
| Urea USP | 10 |
| Deionized water | 61.6 |

### Example 4

Topical lotion composition according to the present invention is prepared from the following components:

| Component | Percent by weight |
|---|---|
| allantoin | 0.2 |
| Carbomer 940 | 0.1 |
| Cetyl alcohol | 2 |
| Dimethicone (Dow fluid 350) | 0.5 |
| Emulsifying wax NF (polawax) | 2 |
| Glyceryl stearate | 2 |
| Hydrogenated polyisobutene | 5 |
| Imidazolidinyl urea | 0.3 |
| Isopropyl myristate | 5 |
| Methyl paraben | 0.2 |
| Propyl paraben | 0.1 |
| Propylene glycol | 5 |
| Putrescine | 1 |
| Silk protein (amino acid) | 1 |
| Tetra sodium EDTA | 0.1 |
| Triethanolamine | 0.3 |
| Deionized water | 75.2 |

### Example 5

Topical composition according to the present invention with lidocaine and putrescine present is prepared from the following components:

| Component | Percent by weight |
|---|---|
| Allantoin | 0.2 |
| Carbomer 940 | 0.4 |
| Carbowax PEG-400 | 5 |
| Imidizolidyl urea | 0.3 |
| Lechithin | 1 |
| Lidocaine | 4 |
| Methyl paraben | 0.2 |
| N-propyl alcohol | 7 |
| Oleic acid | 1 |
| Putrescine | 2 |
| Tetradecyl tetrasodium EDTA | 0.2 |
| Triethanolamine . | 4.75 |
| Urea USP | 10 |
| Deionized water | 63.95 |

### Example 6

Topical massage cream composition according to the present invention is prepared from the following components:

| Component | Percent by weight |
|---|---|
| Arnica oil | 0.5 |
| Calendula oil | 0.5 |
| Camphoracious 804 | 0.05 |
| Cetyl alcohol | 2 |
| Dimethicone (Dow fluid 350) | 0.5 |
| Glyceryl stearate | 3 |
| GMS/PEG 100 stearate | 5 |
| Imidazolidynyl urea | 0.4 |
| Isopropyl palmitate | 5 |
| Lactic acid | 1.25 |
| Menthol USP | 0.05 |
| Mineral oil, medium | 15 |
| Peppermint oil | 0.05 |
| Propylene glycol | 5 |
| Putrescine | 2 |
| Quaternium 15 (Dow 200) | 0.05 |
| Silk protein (amino aicd) | 0.5 |
| Sorbic acid | 0.15 |
| Triethanolamine | 0.2 |
| Urea USP | 1 |
| Deionized water | 57.8 |

### Example 7

Topical cream composition according to the present invention is prepared from the following components:

| Component | Percent by weight |
|---|---|
| Putrescine | 0.8 |
| Glaxo eutectic base | 99.2 |

The putrescine is dissolved in deionized water in a one to one ratio before adding to the Glaxo base.

### Feasibility studies

In order to assess the analgesic effects of the method of use of the compounds of the invention and the compositions of the present invention, studies have been conducted. In the following study, the effect of the composition on a group of subjects in pain was evaluated.

Sixteen subjects experiencing pain were given a sample of the composition of Example 1 (the "example 1" composition) and a sample of the composition of Example 1 that does not include the active ingredient, putrescine (the "placebo" composition). The subjects were advised to topically administer the compositions at different periods of pain and to then rate the effectiveness of the two compositions. Pain was estimated by a ranking system from 0 to 5, wherein 0 was no effect, while 5 represented maximum perceived relief of pain. The data are presented in Table 1 and show that the perceived pain decreased with application of the composition containing putrescine. As well, 15 out of 16 subjects did not perceive a decrease in pain after application of the placebo composition.

**Table 1:**

| Number of subjects reporting the following response | | | | | | |
|---|---|---|---|---|---|---|
| Composition | 0 | 1 | 2 | 3 | 4 | 5 |
| Example 1 | 3 | 1 | - | - | 4 | 8 |
| Placebo | 15 | 1 | - | - | - | - |

Similarly, subjects experiencing pain were given a sample of the composition of Example 1 (the "Example 1" composition) and a sample of the composition of Example 1 that does not include the active ingredient, putrescine (the "placebo" composition). The subjects were advised to administer the compositions at different periods of pain and to then rate the effectiveness of the two compositions. Pain was estimated by a ranking system from 0 to 5, wherein 0 was no effect, while 5 represented maximum perceived relief of pain. The data are presented in Table 2 and show that the perceived pain decreased with application of the composition containing putrescine.

**Table 2**

| Subject No. | Rating of Example 1 | Rating of Placebo | Type of pain |
|---|---|---|---|
| 1 | 4 | 1 | Arthritic pain in back |
| 2 | 4 | 0 | Pain in toe from sports injury |
| 3 | 5 | 0 | Stiffness in knee |
| 4 | 1 | 0 | Tom muscle in leg |
| 5 | 0 | 0 | Soreness from prior ankle injury |
| 6 | 5 | - | Sprained ankle |
| 7 | 5 | 0 | Pain in upper arm |
| 8 | 0 | 0 | Pain associated with circulatory problems in the hands |
| 9 | 5 | 0 | Arthritic pain in toe |
| 10 | 5 | 0 | Arthritic pain in hand |
| 11 | 5 | 0 | Muscle tightness in back |
| 12 | 4 | 0 | Headache |
| 13 | 5 | 0 | Arthritic pain in toe |
| 14 | 3 | 0 | Rotator cuff pain |
| 15 | 5 | 0 | Headache |
| 16 | 0 | 0 | Burning sensation in foot |
| 17 | 5 | 1 | Arthritic pain in back and leg |
| 18 | 5 | 0 | Arthritic wrist |
| 19 | 5 | 0 | Frontal headache |
| 20 | 3 | 1 | Frontal headache |

Tests were also conducted on twelve subjects suffering from arthritis. Half of the subjects were given a placebo for topical administration and the others were given the following composition:

| Component | % w/w |
|---|---|
| deionized water | 46 |
| urea | 20 |
| lactic acid | 7.5 |
| propylene glycol | 5 |
| hydrogenated polyisobutene | 4.5 |
| isopropyl myristate | 4 |
| sodium hydroxide | 3.2 |
| cetyl alcohol | 2.5 |
| peg 100 stearate | 2 |
| putrescine | 2 |
| emulsifying wax | 1.5 |
| silk protein(amino acid) | 1 |
| imidazolidinyl urea | 0.4 |
| allantion | 0.1 |
| carbomer 940 | 0.1 |
| di sodium EDTA | 0.1 |
| quaternium 15 | 0.1 |

This composition contains 2% putrescine. The results are summarized in Table 3. Three people who were given the placebo dropped out prior to completion of the study.

**Table 3**

| Relief Ranking | 2% putrescine Analgesic | Placebo Ranking |
|---|---|---|
| 0 | | 2 |
| 1 | | 1 |
| 2 | | |
| 3 | 2 | |
| 4 | 1 | |
| 5 | 3 | |

Tests were also conducted on people suffering from fibromyalgia to compare relief obtained with a topical composition containing 20 percent triethanolamine salicylate (Bayer Myoflex) to the above-indicated 2 percent putrescine composition. The test was double blind. The results obtained are summarized in Table 4.

**Table 4**

| | | | | |
|---|---|---|---|---|
| Subject | Myoflex Ranking | Duration of Relief (Hours) | 2 Percent Putrescine | Duration of Relief (Hours) |
| 1 | 3 | 1-3 | 4 | 2-12 |
| 2 | 1-2 | 2 | 3-4 | 5-6 |
| 3 | 1 | 2 | 3 | 4-6 |

A useful embodiment of the invention is one in which lidocaine is incorporated into a topical composition:

| Component | Percent by Weight |
|---|---|
| deionized water | 63.95 |
| urea | 10.00 |
| N-propyl alcohol | 7.00 |
| carbowax PEG-400 | 5.00 |
| triethanolamine | 4.75 |
| lidocaine | 4.00 |
| putrescine | 2.00 |
| lechithin | 1.00 |
| oleic acid | 1.00 |
| carbomer 940 | 0.40 |
| imidazolidinyl urea | 0.30 |
| allantion | 0.20 |
| methyl paraben | 0.20 |
| tetradecyl trimethylammonium bromide (Cetrimide) | 0.20 |

## Claims

1. Use of an aliphatic polyamine in the preparation of an analgesic medicament for topical treatment of a site of local discomfort.

2. Use of an aliphatic polyamine in the preparation of a medicament for topical treatment of nociceptive stimulation.

3. Use according to claim 1 or 2, wherein the polyamine is an alkylamine, preferably in which all of the amines are primary alkyl amines.

4. Use according to claim 1, 2 or 3, wherein the polyamine has up to fifteen carbon atoms.

5. Use according to any of claims 1 to 4, wherein the polyamine has up to four amino groups, optionally wherein the polyamine has up to three amino groups, and/or wherein the polyamine is saturated, and/or wherein the polyamine is non-cyclic, and/or wherein the polyamine has up to ten carbon atoms.

6. Use according to claim 5, wherein the polyamine is spermine or putrescine or spermidine or cadaverine or any combination thereof.

7. Use according to claim 5, wherein the polyamine has at least four carbon atoms, and/or wherein the polyamine has up to seven carbon atoms.

8. Use according to any preceding claim wherein the polyamine is present, at least in part, as a pharmaceutically acceptable salt.

9. Use according to claim 8, wherein the polyamine makes up between 0.1 and 10 weight percent of the composition administered to the site, or between 0.2 and 9 percent, or between 0.3 and 8 percent, or between 0.4 and 7 percent, or between 0.5 and 6 percent, or between 0.6 and 5 percent, or between 0.7 and 5 percent, or between 0.8 and 5 percent, or between 1 and 4 percent or between 1 and 3 percent, or between 1 and 2 percent.

10. Use according to any preceding claim, wherein the medicament is for topical administration to a site located on the epidermis of a human.

11. Use according to any of claims 8 to 10, wherein the composition further comprises a chaotropic agent, preferably wherein the chaotropic agent is urea, or is oleic acid, or is a combination of urea and oleic acid.

12. Use according to claim 11, wherein urea makes up about, or at least 5 percent, or about or at least 10 percent, or about at least 15 percent, or about or at least 20 percent, or about or at least 25 percent, or about or at least 30 percent, or about or at least 35 percent, or about or at least 40 percent, by weight, of the composition.

13. Use according to any of claims 8 to 12, wherein the composition includes a pharmaceutically acceptable vehicle compatible with mammalian skin, and particularly, human skin, preferably wherein the composition is a cream, or a is a spray, or is an ointment or is a gel, or is a lotion.

14. Use according to any of claims 8 to 13, wherein the composition further comprises beta 1,3-D glucan, and/or wherein the composition further comprises lidocaine.

15. Use according to any preceding claim, wherein the medicament is for the treatment of discomfort that is the result of a sports injury, a physical assault, arthritis, rheumatism, headache, shingles, surgical pain, or a combination of any of the foregoing.

16. Use according to any of claims 1 to 15, wherein the polyamine is incorporated into a patch, preferably wherein the release of the polyamine from the patch is controlled to produce the analgesia over a period of time, wherein the period of time can be up to about one week, or wherein the period of time is between about one hour and one week, or between one hour and six days, or between six hours and five days, or between six hours and four days, or between twenty-four hours and three days, or between one day and two days.

17. Use according to any preceding claim, wherein the medicament is for application to a site that is free of extracellular wound scar skin tissue, and/or wherein the medicament is for treatment of a site that is free of a wound undergoing healing of the skin.

18. A topical analgesic drug composition comprising an analgesia-inducing amount of at least one aliphatic polyamine and urea, wherein the urea is present in the amount of at least about 5, or about 10, or about 15 percent urea, or about 20 percent urea, or about 25 percent urea, or about 30 percent urea, or about 35 percent urea, or about 40 percent urea.

19. A topical analgesic drug composition according to claim 18, and further according to any of claims 3 to 9, and any combination of the elements defined therein.

20. A topical analgesic drug composition according to claim 18 or 19, and further comprising a pharmaceutically acceptable vehicle compatible with mammalian skin, and particularly, human skin.

21. A topical analgesic drug composition according to any of claims 18 to 20, wherein the composition is a cream, or a is a spray, or is an ointment or is a gel, or is a lotion, and/or wherein the composition further comprises beta 1,3-D glucan, and/or wherein the composition further comprises lidocaine.

22. A topical analgesic drug composition according to any of claims 18, 19, 20 and 21, wherein the polyamine is incorporated into a patch.

23. A topical analgesic drug composition according to any one of claims 18 to 22, wherein the polyamine is present in an amount between 0.1 and 10 wt.% of the composition.

## Patentansprüche

1. Verwendung eines aliphatischen Polyamins in der Herstellung eines analgetischen Medikaments zur topischen Behandlung einer Stelle lokaler Beschwerden.

2. Verwendung eines aliphatischen Polyamins in der Herstellung eines Medikaments zur topischen Behandlung von nozizeptiver Stimulation.

3. Verwendung gemäß Anspruch 1 oder 2, wobei das Polyamin ein Alkylamin ist, in welchem vorzugsweise alle Amine primäre Alkylamine sind.

4. Verwendung gemäß Anspruch 1, 2 oder 3, wobei das Polyamin bis zu fünfzehn Kohlenstoffatome besitzt.

5. Verwendung gemäß einem der Ansprüche 1 bis 4, wobei das Polyamin bis zu vier Aminogruppen besitzt, wobei das Polyamin gegebenenfalls bis zu drei Aminogruppen besitzt, und/oder wobei das Polyamin gesättigt ist und/oder wobei das Polyamin nichtcyclisch ist, und/oder wobei das Polyamin bis zu zehn Kohlenstoffatome besitzt.

6. Verwendung gemäß Anspruch 5, wobei das Polyamin Spermin oder Putrescin oder Spermidin oder Cadaverin oder eine Kombination davon ist.

7. Verwendung gemäß Anspruch 5, wobei das Polyamin mindestens vier Kohlenstoffatome besitzt, und/oder wobei das Polyamin bis zu sieben Kohlenstoffatome besitzt.

8. Verwendung gemäß einem der vorhergehenden Ansprüche, wobei das Polyamin, mindestens zum Teil, als pharmazeutisch verträgliches Salz vorliegt.

9. Verwendung gemäß Anspruch 8, wobei das Polyamin zwischen 0,1 und 10 Gewichtsprozent der auf die Stelle verabreichten Zusammensetzung ausmacht, oder zwischen 0,2 und 9 Prozent, oder zwischen 0,3 und 8 Prozent, oder zwischen 0,4 und 7 Prozent, oder zwischen 0,5 und 6 Prozent, oder zwischen 0,6 und 5 Prozent, oder zwischen 0,7 und 5 Prozent, oder zwischen 0,8 und 5 Prozent, oder zwischen 1 und 4 Prozent, oder zwischen 1 und 3 Prozent, oder zwischen 1 und 2 Prozent.

10. Verwendung gemäß einem der vorhergehenden Ansprüche, wobei das Medikament zur topischen Verabreichung auf eine auf der Epidermis eines Menschen gelegene Stelle dient.

11. Verwendung gemäß einem der Ansprüche 8 bis 10, wobei die Zusammensetzung des Weiteren ein chaotropes Mittel umfasst, wobei vorzugsweise das chaotrope Mittel Harnstoff, oder Ölsäure, oder eine Kombination von Harnstoff und Ölsäure ist.

12. Verwendung gemäß Anspruch 11, wobei Harnstoff etwa oder mindestens 5 Gewichtsprozent, oder etwa oder mindestens 10 Gewichtsprozent, oder etwa mindestens 15 Gewichtsprozent, oder etwa oder mindestens 20 Gewichtsprozent, oder etwa oder mindestens 25 Gewichtsprozent, oder etwa oder mindestens 30 Gewichtsprozent, oder etwa oder mindestens 35 Gewichtsprozent, oder etwa oder mindestens 40 Gewichtsprozent der Zusammensetzung ausmacht.

13. Verwendung gemäß einem der Ansprüche 8 bis 12, wobei die Zusammensetzung einen pharmazeutisch verträglichen, mit Säugetierhaut und insbesondere Menschenhaut kompatiblen Schlepper einschließt, wobei die Zusammensetzung vorzugsweise eine Creme, oder ein Spray, oder eine Salbe, oder ein Gel, oder eine Lotion ist.

14. Verwendung gemäß einem der Ansprüche 8 bis 13, wobei die Zusammensetzung des Weiteren beta-1,3-D-Glucan umfasst, und/oder wobei die Zusammensetzung des Weiteren Lidocain umfasst.

15. Verwendung gemäß einem der vorhergehenden Ansprüche, wobei das Medikament zur Behandlung von Beschwerden dient, welche das Resultat einer Sportverletzung, eines physischen Angriffs, von Arthritis, Rheumatismus, Kopfschmerz, einer Gürtelrose, von Operationsschmerzen, oder einer Kombination jedweder der vorgenannten sind.

16. Verwendung gemäß einem der Ansprüche 1 bis 15, wobei das Polyamin in ein Pflaster eingebracht ist, wobei vorzugsweise die Freisetzung des Polyamins aus dem Pflaster eingestellt ist, die Analgesie über einen Zeitraum zu erzeugen, wobei der Zeitraum bis zu etwa einer Woche sein kann, oder wobei der Zeitraum zwischen etwa einer Stunde und einer Woche ist, oder zwischen einer Stunde und sechs Tagen, oder zwischen sechs Stunden und fünf Tagen, oder zwischen sechs Stunden und vier Tagen, oder zwischen vierundzwanzig Stunden und drei Tagen, oder zwischen einem Tag und zwei Tagen.

17. Verwendung gemäß einem der vorhergehenden Ansprüche, wobei das Medikament zum Auftragen auf eine Stelle dient, welche frei von extrazellulärem Wundnarben-Hautgewebe ist, und/oder wobei das Medikament zur Behandlung einer Stelle dient, welche frei von einer Wunde, welche Heilung der Haut durchläuft, ist.

18. Topische analgetische Arzneizusammensetzung, umfassend eine Analgesie erzeugende Menge mindestens eines aliphatischen Polyamins und Harnstoff, wobei der Harnstoff in der Menge von mindestens etwa 5, oder etwa 10, oder etwa 15 Prozent Harnstoff, oder etwa 20 Prozent Harnstoff, oder etwa 25 Prozent Harnstoff, oder etwa 30 Prozent Harnstoff, oder etwa 35 Prozent Harnstoff, oder etwa 40 Prozent Harnstoff vorhanden ist.

19. Topische analgetische Arzneizusammensetzung gemäß Anspruch 18, und des Weiteren gemäß einem der Ansprüche 3 bis 9, und jedwede Kombination der darin definierten Elemente.

20. Topische analgetische Arzneizusammensetzung gemäß Anspruch 18 oder 19, des Weiteren umfassend einen pharmazeutisch verträglichen, mit Säugetierhaut und insbesondere Menschenhaut kompatiblen Schlepper.

21. Topische analgetische Arzneizusammensetzung gemäß einem der Ansprüche 18 bis 20, wobei die Zusammensetzung eine Creme, oder ein Spray, oder eine Salbe, oder ein Gel, oder eine Lotion ist, und/oder wobei die Zusammensetzung des Weiteren beta-1,3-D-Glucan umfasst, und/oder wobei die Zusammensetzung des Weiteren Lidocain umfasst.

22. Topische analgetische Arzneizusammensetzung gemäß einem der Ansprüche 18, 19, 20 und 21, wobei das Polyamin in ein Pflaster eingebracht ist.

23. Topische analgetische Arzneizusammensetzung gemäß einem der Ansprüche 18 bis 22, wobei das Polyamin in einer Menge zwischen 0,1 und 10 Gewichtsprozent der Zusammensetzung vorhanden ist.

## Revendications

1. Utilisation d'une polyamine aliphatique dans la préparation d'un médicament analgésique pour le traitement topique d'un site d'inconfort local.

2. Utilisation d'une polyamine aliphatique dans la préparation d'un médicament pour le traitement topique d'une stimulation nociceptive.

3. Utilisation selon la revendication 1 ou 2, dans laquelle la polyamine est une alkylamine, de préférence dans laquelle toutes les amines sont des alkyl amines primaires.

4. Utilisation selon la revendication 1, 2 ou 3, dans laquelle la polyamine a jusqu'à quinze atomes de carbone.

5. Utilisation selon l'une quelconque des revendications 1 à 4, dans laquelle la polyamine a jusqu'à quatre groupes amino, facultativement dans laquelle la polyamine a jusqu'à trois groupes amino, et/ou dans laquelle la polyamine est saturée et/ou dans laquelle la polyamine est non cyclique et/ou dans laquelle la polyamine a jusqu'à dix atomes de carbone.

6. Utilisation selon la revendication 5, dans laquelle la polyamine est la spermine ou la putrescine ou la spermidine ou la cadavérine ou toute combinaison de celles-ci.

7. Utilisation selon la revendication 5, dans laquelle la polyamine a au moins quatre atomes de carbone, et/ou dans laquelle la polyamine a jusqu'à sept atomes de carbone.

8. Utilisation selon l'une quelconque des revendications précédentes, dans laquelle la polyamine est présente, au moins en partie, sous la forme d'un sel pharmaceutiquement acceptable.

9. Utilisation selon la revendication 8, dans laquelle la polyamine représente entre 0,1 et 10 pour cent en poids de la composition administrée au site, ou entre 0,2 et 9 pour cent, ou entre 0,3 et 8 pour cent, ou entre 0,4 et 7 pour cent, ou entre 0,5 et 6 pour cent, ou entre 0,6 et 5 pour cent, ou entre 0,7 et 5 pour cent, ou entre 0,8 et 5 pour cent, ou entre 1 et 4 pour cent, ou entre 1 et 3 pour cent, ou entre 1 et 2 pour cent.

10. Utilisation selon l'une quelconque des revendications précédentes, dans laquelle le médicament est pour l'administration topique à un site se trouvant sur l'épiderme d'un humain.

11. Utilisation selon l'une quelconque des revendications 8 à 10, dans laquelle la composition comprend en outre un agent chaotropique, de préférence dans laquelle l'agent chaotropique est l'urée, ou est l'acide oléique, ou est une combinaison d'urée et d'acide oléique.

12. Utilisation selon la revendication 11, dans laquelle l'urée représente environ ou au moins 5 pour cent, ou environ ou au moins 10 pour cent, ou environ au moins 15 pour cent, ou environ ou au moins 20 pour cent, ou environ ou au moins 25 pour cent, ou environ ou au moins 30 pour cent, ou environ ou au moins 35 pour cent, ou environ ou au moins 40 pour cent, en poids, de la composition.

13. Utilisation selon l'une quelconque des revendications 8 à 12, dans laquelle la composition inclut un véhicule pharmaceutiquement acceptable compatible avec la peau de mammifère, et en particulier la peau humaine, de préférence dans laquelle la composition est une crème, ou est un pulvérisateur, ou est une pommade ou est un gel, ou est une lotion.

14. Utilisation selon l'une quelconque des revendications 8 à 13, dans laquelle la composition comprend en outre du β 1,3-D glucane et/ou dans laquelle la composition comprend en outre de la lidocaïne.

15. Utilisation selon l'une quelconque des revendications précédentes, dans laquelle le médicament est pour le traitement d'un inconfort qui est le résultat d'une blessure du sport, d'une agression physique, d'une arthrite, d'un rhumatisme, d'une céphalée, d'un zona, d'une douleur chirurgicale, ou toute combinaison des inconforts précités.

16. Utilisation selon l'une quelconque des revendications 1 à 15, dans laquelle la polyamine est incorporée dans un timbre, de préférence dans laquelle la libération de la polyamine à partir du timbre est contrôlée pour produire l'analgésie sur une période de temps, dans laquelle la période de temps peut aller jusqu'à environ une semaine, ou dans laquelle la période de temps est entre environ une heure et une semaine, ou entre une heure et six jours, ou entre six heures et cinq jours, ou entre six heures et quatre jours, ou entre vingt-quatre heures et trois jours, ou entre un jour et deux jours.

17. Utilisation selon l'une quelconque des revendications précédentes, dans laquelle le médicament est pour l'application à un site qui est exempt de tissu cutané présentant des cicatrices et plaies extracellulaire, et/ou dans laquelle le médicament est pour le traitement d'un site qui est exempt d'une plaie de la peau en cours de cicatrisation.

18. Composition médicamenteuse analgésique topique comprenant une quantité, induisant une analgésie, d'au moins une polyamine aliphatique et d'urée, dans laquelle l'urée est présente en la quantité d'au moins environ 5, ou environ 10, ou environ 15 pour cent d'urée, ou environ 20 pour cent d'urée, ou environ 25 pour cent d'urée, ou environ 30 pour cent d'urée, ou environ 35 pour cent d'urée, ou environ 40 pour cent d'urée.

19. Composition médicamenteuse analgésique topique selon la revendication 18, ainsi que selon l'une quelconque des revendications 3 à 9, et toute combinaison des éléments qui y sont définis.

20. Composition médicamenteuse analgésique topique selon la revendication 18 ou 19, et comprenant en outre un véhicule pharmaceutiquement acceptable compatible avec la peau de mammifère, et en particulier la peau humaine.

21. Composition médicamenteuse analgésique topique selon l'une quelconque des revendications 18 à 20, la composition étant une crème, ou étant un pulvérisateur, ou étant une pommade ou étant un gel, ou étant une lotion, et/ou la composition comprenant en outre du β 1,3-D glucane, et/ou la composition comprenant en outre de la lidocaïne.

22. Composition médicamenteuse analgésique topique selon l'une quelconque des revendications 18, 19, 20 et 21, dans laquelle la polyamine est incorporée dans un timbre.

23. Composition médicamenteuse analgésique topique selon l'une quelconque des revendications 18 à 22, dans laquelle la polyamine est présente en une quantité entre 0,1 et 10 % en poids de la composition.
